# EUROPÄISCHE PATENTSCHRIFT

(11) **EP 1 345 569 B1**
(45) Veröffentlichungstag und Bekanntmachung des Hinweises auf die Patenterteilung: **11.11.2009**
(21) Anmeldenummer: 01989613.3
(22) Anmeldetag: 21.12.2001
(51) Int. Cl.: A61K 8/86, A61Q 1/04, A61Q 11/00, A61Q 19/00

(54) **KOSMETISCHE PRÄPARATE UND ANWENDUNGEN, ENTHALTEND POLYETHYLENWACHSE MIT VERBESSERTEN ORGANOLEPTISCHEN EIGENSCHAFTEN**
COSMETIC PREPARATIONS AND USE THEREOF, CONTAINING POLYETHYLENE WAX WITH IMPROVED ORGANOLEPTIC PROPERTIES
PREPARATIONS COSMETIQUES, ET LEUR UTILISATION, CONTENANT DES CIRES DE POLYETHYLENE AYANT DES PROPRIETES ORGANOLEPTIQUES AMELIOREES

(30) Priorität: 22.12.2000 DE 10064799; 22.12.2000 DE 10064800
(43) Veröffentlichungstag der Anmeldung: 24.09.2003
(73) Patentinhaber: BASF SE, 67056 Ludwigshafen (DE)
(72) Erfinder: WITTKOWSKI, Lars, 68167 Mannheim (DE); ZEITZ, Katrin, 67067 Ludwigshafen (DE); WEBER, Wilhelm, 67435 Neustadt (DE); DECKERS, Andreas, 55234 Flomborn (DE)
(86) Internationale Anmeldenummer: PCT/EP2001/015239
(87) Internationale Veröffentlichungsnummer: WO 2002/051360

(56) Entgegenhaltungen:
- WO-A-99/09937
- WO-A-02/051885
- WO-A-02/059166
- US-A- 4 534 963
- US-A- 5 556 613
- US-A- 5 746 812
- DATABASE CA [Online] CHEMICAL ABSTRACTS SERVICE, COLUMBUS, OHIO, US; IMAI, TAKEO ET AL: "Oily solids containing modified polyethylene waxes for cosmetics" retrieved from STN Database accession no. 123:65584 XP002219657 & JP 07 118122 A (KAO CORP, JAPAN) 9. Mai 1995 (1995-05-09)
- LINDA COFFIN: "Toiletries Library: All About Waxes" [Online] XP002219656 Gefunden im Internet: <URL: http://www.luxurylane.com/thelibrary/refer ence/waxes.htm> [gefunden am 2002-11-06] siehe Absatz "Poylethylene Waxes" auf Seite 6/10

## Beschreibung

Die vorliegende Erfindung betrifft kosmetische Präparate und Anwendungen, enthaltend Polyethylenwachse mit verbesserten organoleptischen Eigenschaften, hergestellt durch (Co-)Polymerisation von Ethylen unter Hochdruckbedingungen unter Verwendung eines aliphatischen oder alicyclischen Ketons als Molekulargewichtsregler, sowie ein Verfahren zur Herstellung von kosmetischen Präparaten und Anwendungen mit verbesserten organoleptischen Eigenschaften. Weiterhin betrifft die vorliegende Erfindung die Verwendung von Polyethylenwachsen mit verbesserten organoleptischen Eigenschaften für kosmetische Präparate und Anwendungen, beispielsweise für dekorative Kosmetik, pflegende und reinigende Kosmetik sowie pharmazeutische Zubereitungen oder Hygieneartikel. Insbesondere betrifft die vorliegende Erfindung mikronisierte Polyethylenwachse; hergestellt durch (Co-)Polymerisation von Ethylen unter Hochdruckbedingungen unter Verwendung eines aliphatischen oder alicyclischen Ketons als Molekulargewichtsreglers und anschließende Mikronisierung, sowie ihre Verwendung für kosmetische Präparate und als Träger für Zahnpasta.

Polyethylenwachse werden in zahlreichen Kosmetika eingesetzt. An die zu verwendenden Wachse werden anspruchsvolle Forderungen gestellt, was ihre organoleptischen Eigenschaften betrifft. So dürfen die Wachse keinesfalls unangenehme Gerüche verströmen, weil sie sonst in kosmetischen Präparaten unverkäuflich wären. Aus dem gleichen Grund müssen sie geschmacksneutral sein. Weiterhin müssen sie gute Verarbeitungseigenschaften aufweisen. Schließlich müssen sie wirtschaftlich vorteilhaft sein, d.h. sie müssen sich leicht und mit hoher Ausbeute herstellen lassen. Schlüsselparameter sind das mittlere Molekulargewicht, die Molekulargewichtsverteilung und dabei insbesondere die Breite der Molekulargewichtsverteilung sowie die hochmolekularen und niedermolekularen Anteile, gegebenenfalls eingebaute Comonomere, Länge und Verteilung der Verzweigungen sowie Verunreinigungen des Polymers, beispielsweise durch flüchtige Oligomere, Restmonomere sowie Zersetzungsprodukte von Katalysatoren bzw. Radikalstartern und Molekulargewichtsreglern.

Diese Schlüsselparameter sind besonders wichtig in Anwendungen, in denen Polyethylenwachse in mikronisierter Form eingesetzt werden. Unter mikronisierten Polyethylenwachsen werden im Folgenden solche Wachse verstanden, die man durch Vermahlen oder Versprühen in die geeignete Morphologie gebracht hat, d.h. Wachs-Pulver mit einem maximalen Partikeldurchmesser von 100 µm. Eine neuere Anwendung für die chemisch weitgehend inerten Polyethylenwachse sind Zahnpasten, in denen sie als wenig abrasiv wirkendes Trägermaterial das sonst zugesetzte Kieselgel ersetzen können.Gerade'in modernen Zahnpasten wird häufig auf Polyethylenwachs als Träger statt Kieselgel oder Calciumcarbonat zurückgegriffen, weil Polyethylenwachs-haltige Zahnpasten deutlich schwächer abrasiv wirken und deshalb der Zahnschmelz auch bei häufigem Putzen nicht so stark angegriffen wird wie bei der Verwendung von Kieselgel.

In US 4,534,963 werden kosmetische Präparate offengelegt, die mikronisierte Polyethylenwachse in einem Anteil von 0.1 bis 15 Gew.-% als Bindemittel enthalten.

Polyethylenwachse lassen sich nach verschiedenen verfahren herstellen, die sich grob in Niederdruckverfahren, durchgeführt bei 20 bis 100 bar, und Hochdruckverfahren, durchgeführt bei 500 bis 4000 bar, einteilen lassen. Das Hochdruckverfahren ist ein radikalisches Polymerisationsverfahren, das im Allgemeinen ohne Katalysator auskommt (vgl. beispielsweise: Ullmann's Enyclopädie der technischen Chemie*, 4. Auflage, Stichworte: Wachse, Bd. 24, S. 36 ff., Thieme Verlag Stuttgart, 1977). Zum Starten der radikalischen Kettenreaktion verwendet man meistens ein oder mehrere organische Peroxide, beispielsweise die Trigonox® oder Perkadox®-Marken der Akzo Nobel, oder aber Luft bzw. Luftsauerstoff. Der billigste und deshalb am weitesten verbreitete Radikalstarter ist Luft bzw. Luftsauerstoff.

Zur Einstellung des geeigneten Molekulargewichts verwendet man als Molekulargewichtsregler oder kurz Regler bezeichnete Substanzen. Bei der Verwendung eines Stoffes als Regler ist zu beachten, dass er hinreichend effizient ist, weil die Dosierung sehr großer Mengen an Reglern unwirtschaftlich ist.

Ein häufig verwendeter Regler ist Wasserstoff, der aber bei der Verwendung von Luft oder Luftsauerstoff als Radikalstarter zur Bildung von Knallgas führen kann und deshalb aus sicherheitstechnischen Gründen Bedenken hervorruft.

Weitere häufig verwendete Regler sind Kohlenmonoxid CO und Alkane wie beispielsweise Ethan oder Propan. Kohlenmonoxid ist stark giftig, so dass bei der Verwendung aufwendige Sicherheitsmaßnahmen erforderlich sind. Gasförmige Regler wie Ethan und Propan erfordern ebenfalls strenge Sicherheitsregeln.

Die häufig verwendeten Aldehyde, beispielsweise Propionaldehyd, führen zu Wachsen, die einen charakteristischen unangenehmen Geruch aufweisen und als Komponente für kosmetische Anwendungen wie beispielsweise Lippenstifte, Lidschatten oder Rouge nur nach aufwändiger Verarbeitung geeignet sind. Selbst nach intensiver Bear-beitung mit Wasserdampf lässt sich noch ein schwacher, aber unangenehmer Geruch feststellen.

Die Verwendung von Ketonen als Molekulargewichtsregler bei der Herstellung von LDPE ist bereits bekannt. In EP-A 0 928 797 wird ein Verfahren unter Verwendung von Methylethylketon als Regler vorgeschlagen, mit Hilfe dessen ein LDPE hergestellt wird, das sich für Extrusionsprodukte eignet, beispielsweise für Folien mit gutem Durchstichwiderstand, aber nicht für Wachse.

In DE-A 1 908 964 wird ein Verfahren offengelegt, durch das sich Ethylenhomopolymerisate im Hochdruckverfahren herstellen lassen. Kennzeichnend an dem beschriebenen Verfahren ist die Verwendung eines peroxidischen Radikalstarters in der ersten Reaktionszone und Luft in der zweiten. Als Regler werden Propionaldehyd oder Methylethylketon empfohlen. Man erhält ein hochmolekulares Polyethylen, welches sich besonders gut zur Herstellung hochtransparenter Feinfolien oder widerstandsfähiger Verpackungsfolien eignet.

In US 3,334,081 wird ein Hochdruck-Polymerisationsverfahren mit erhöhtem Umsatz beschrieben, das auf der Einspeisung von Ethylen an mindestens zwei verschiedenen Stellen des Reaktors beruht. Als Radikalstarter werden eine Vielzahl von organischen Peroxiden und als Regler eine Vielzahl organischer Verbindungen, bevorzugt Ketone wie beispielsweise Methylethylketon, empfohlen. Nachteilig an dem beschriebenen Verfahren ist jedoch der hohe Investitionsaufwand, der auf den zahlreichen Dosierstellen beruht, die alle extrem druckstabil und dicht ausgelegt werden müssen. Dadurch wird der Investitionsbedarf für eine Polymerisationsanlage sehr hoch.

In US 3,317,504 wird ein Verfahren zur Herstellung von Polyethylen, das zur Herstellung von Filmen geeignet ist, offengelegt, welches eine spezielle Temperaturführung und die Verwendung spezieller Molekulargewichtsregler, beispielsweise Methylethylketon, erfordert. Beiden offenbarten Verfahrensparametern werden jedoch keine Wachse erhalten.

Im rumänischen Patent RO 75,587 (Priorität: 18.04.1979, aus CA 96: 200372s) wird die Herstellung geruchloser LDPE-Sorten beschrieben. Als Regler wird eine Mischung von Methylvinylketon mit Propan, Ethan und CO verwendet, zum Starten der Reaktion eine Mischung verschiedener organischer Peroxide. Die Verwendung von CO ist aber wegen der starken Giftigkeit von Nachteil, weil die Rohre und der Reaktorausgang speziell gegen Entweichen von CO gesichert werden müssen.

Grundsätzlich lassen sich Polyethylenwachse auch im Niederdruckverfahren herstellen. Dazu ist im Allgemeinen ein Katalysator erforderlich, beispielsweise ein Ziegler-Natta-Katalysator wie in US 3,129,211, oder ein Metallocen-Katalysator wie in EP-A 0 890 619. Die organoleptischen Eigenschaften mikronisierter Polyethylenwachse, hergestellt nach einem Niederdruckverfahren, sind jedoch in Anwendungen wie kosmetischen Präparaten noch zu verbessern.

Es bestand die Aufgabe, Polyethylenwachse in mikronisierter oder nicht-mikronisierter Form zur Verwendung in Kosmetika, kosmetischen Zubereitungen oder Zahnpasta bereitzustellen, die
- wirtschaftlich vorteilhaft herstellbar sind,
- keine Verwendung von extrem explosionsgefährlichen Reglern wie Wasserstoff oder hochgiftigen Reglern wie Kohlenmonoxid verlangen,
- gute organoleptische Eigenschaften haben, d.h. geruchlos und geschmacklos sind,
- leicht weiterzuverarbeiten sind
- und sich gut zur Herstellung von kosmetischen Zubereitungen eignen.

Es wurde nun gefunden, dass sie die Aufgaben dadurch lösen lassen, dass man mit speziellen Ketonen hergestellte Polyethylenwachse verwendet.

Die Polyethylenwachse werden durch Polymerisation oder Copolymerisation von Ethylen in Hochdruckautoklaven oder in Rohrreaktoren hergestellt. Hochdruckautoklaven sind in sogenannten gedrungenen oder langgestreckten Ausführungsformen bekannt. Die bekannten Rohrreaktoren (Ullmanns Encyclopädie der technischen Chemie, Band 19, S. 169 und S. 173 ff (1980), Verlag Chemie Weinheim, Deerfield Beach, Basel sowie Ullmann's Enyclopädie der technischen Chemie, 4. Auflage, Stichworte: wachse, Bd. 24, S. 36 ff., Thieme Verlag Stuttgart, 1977) zeichnen sich durch einfache Handhabung und geringe Wartung aus und sind gegenüber gerührten Autoklaven von Vorteil. Die Polymerisation oder Copolymerisation erfolgt üblicherweise bei Temperaturen von 400 bis 4000 bar, bevorzugt von 500 bis 5000 bar und besonders bevorzugt 1000 bis 3500 bar.

Die Reaktionstemperatur beträgt 180 bis 350°C, bevorzugt sind 200 bis 320°C.

Es lassen sich auch Copolymerisate mit Ethylen herstellen, wobei prinzipiell alle radikalisch mit Ethylen copolymerisierbaren Olefine als Comonomere geeignet sind. Bevorzugt sind
- 1-Olefine wie Propylen, 1-Buten, 1-Penten, 1-Hexen, 1-Octen und 1-Decen,
- Acrylate wie Acrylsäure, Acrylsäuremethylester, Acrylsäureethylester, Acrylsäure-n-butylester, Acrylsäure-2-ethylhexylester oder Acrylsäure-tert.-butylester;
- Methacrylsäure, Methacrylsäuremethylester, Methacrylsäureethylester, Methacrylsäure-n-butylester oder Methacrylsäuretert.-butylester;
- Vinylcarboxylate, wobei Vinylacetat besonders bevorzugt ist,
- Ungesättigte Dicarbonsäuren, besonders bevorzugt ist Maleinsäure,
- ungesättigte Dicarbonsäurederivate, besonders bevorzugt sind Maleinsäureanhydrid und Maleinsäurealkylimide wie beispielsweise Maleinsäuremethylimid.

Der Comonomeranteil beträgt maximal 50 mol-%, bevorzugt maximal 20 mol-%.

Es wurde nun gefunden, dass sich die oben beschriebenen Polyethylenwachse mit verbesserten organoleptischen Eigenschaften am besten herstellen lassen, wenn man während der Polymerisation als Regler ein oder mehrere aliphatische oder alicyclische Ketone der allgemeinen Formeln I 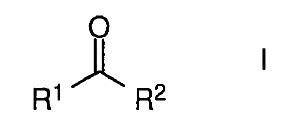 nimmt.

Dabei sind die Reste R¹ und R² gleich oder verschieden und ausgewählt aus
- C₁-C₆-Alkyl wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, iso-Amyl, n-Hexyl, iso-Hexyl, sec.-Hexyl, besonders bevorzugt C₁-C₄-Alkyl wie Methyl, Ethyl, n-Propyl, iso-Propyl, n-Butyl, iso-Butyl, sec.-Butyl und tert.-Butyl;
- C₃-C₁₂-Cycloalkyl wie Cyclopropyl, Cyclobutyl, Cyclopentyl, Cyclohexyl, Cycloheptyl, Cyclooctyl, Cyclononyl, Cyclodecyl, Cycloundecyl und Cyclododecyl; bevorzugt sind Cyclopentyl, Cyclohexyl und Cycloheptyl;

In einer weiteren Ausführungsform sind die Reste R¹ und R² miteinander kovalent verbunden. So können R¹ und R² beispielsweise gemeinsam sein: - (CH₂) ₄-, -(CH₂) ₅-, -(CH₂) ₆, -(CH₂)₇-, -CH (CH₃) -CH₂-CH₂-CH (CH₃) - oder - CH (CH₃)-CH₂-CH₂-CH₂-CH(CH₃) -.

Bevorzugte Beispiele sind Aceton, Methylethylketon "MEK", Methylisobutylketon "MIBK", 2-Pentanon, 3-Pentanon oder Cyclopentanon, Cyclohexanon oder Cycloheptanon. Besonders bevorzugte Beispiele sind Aceton, Methylethylketon, Methylisobutylketon und Cyclohexanon; ganz besonders bevorzugt sind Methylethylketon und vor allem Cyclohexanon. Unter diesen Bedingungen lässt sich auf die Verwendung von CO, Ethan und Propan als Regler verzichten.

Nach der Herstellung lässt sich das in großtechnischen Anlagen üblicherweise als Granulat, Grieß oder Pulver anfallende Wachs sofort in den entsprechenden kosmetischen Anwendungen verwenden.

Die oben beschriebenen Polyethylenwachse haben eine Dichte von 0,8 bis 1,0 g/cm³, bevorzugt von 0,90 bis 0,96 g/cm³ und besonders bevorzugt von 0,93 bis 0,95 g/cm³, gemessen bei 23°C. Die Schmelzviskositäten liegen im Bereich von 20 bis 20000 cSt, bevorzugt im Bereich von 800 bis 2000 cSt, gemessen bei 120°C, das entspricht einem Molekulargewicht M_{w} von maximal 40.000 g, bevorzugt maximal 10.000 g und besonders bevorzugt maximal 7500 g. Die Molekulargewichtsverteilung liegt im Bereich von 2 bis 10. Die Schmelzpunkte liegen im Bereich von 60 bis 125°C, bevorzugt 80 bis 120°C.

Die Wachse zeigen sehr gute organoleptische Eigenschaften, insbesondere sind sie Geruchs- und Geschmacks-neutral. Das ist insofern überraschend, weil sowohl die verwendeten Ketone und insbesondere Methylethylketon als auch die daraus unter Hochdruckbedingungen leicht entstehenden Dimere, Trimere und ähnliche Produkte einen charakteristischen und keinesfalls angenehmen Eigengeruch aufweisen.

Die Messung der organoleptischen Eigenschaften lässt sich apparativ, beispielsweise durch Gaschromatographie oder Differentialthermogravimetrie, bestimmen, wobei durch getrennte oder hintereinandergeschaltete Messapparaturen die Menge und die Art der entweichenden flüchtigen Verbindungen ermittelt wird. Von hoher Signifikanz sind die Tests durch Probandenteams.

Aufgrund ihrer sehr guten organoleptischen Eigenschaften eignen sich die oben beschriebenen Wachse vorzüglich für Präparate und Anwendungen der dekorativen Kosmetik, ausgewählt aus Lippenstiften, Gesichtspuder, Blushes, Lidschatten, Lidstrichstiften, Grundierungen, Make-up-Zubereitungen, Wimperntusche und Augenbrauenstiften. Ein Gegenstand der vorliegenden Erfindung ist daher die Verwendung der beschriebenen Polyethylenwachse für Präparate und Anwendungen der dekorativen Kosmetik, ausgewählt aus Lippenstiften, Gesichtspuder, Blushes, Lidschatten, Lidstrichstiften, Grundierungen, Make-up-Zubereitungen, Wimperntusche und Augenbrauenstiften.

Ein weiterer Gegenstand der vorliegenden Erfindung ist die Verwendung der oben beschriebenen Wachse für Präparate und Anwendungen der pflegenden und reinigenden Kosmetik, ausgewählt aus Reinigungslotions, Handcremes, Handwaschpasten, Hautpflegemitteln, Intimpflegemitteln, Fußpflegemitteln, Lichtschutzmitteln, Repellents, Rasiermitteln, Haarentfernungsmitteln, Antiaknemitteln, Make-up, Nagelpflegemitteln, Kajalstiften, Seifen, Syndets, flüssigen Wasch-, Dusch-, und Badepräparaten, Cold Creams, Hautpflegeprodukte, Exfoliating Scrub Soaps, Gesichts-, Hand- oder Fuß-Peelings, Body-Scrubs, Gele und Lotionen.

Die oben beschriebenen Polyethylenwachse lassen sich hervorragend konfektionieren und so in die zum Einsatz erforderliche oder gut geeignete Darreichungsform bringen. So sind verschiedene Korngrößen beispielsweise durch Sprühen erhältlich.

Außerdem können die Wachse in verschiedenster Weise granuliert werden. Die Methoden der Granulierung von Polyethylenwachsen sind an sich bekannt. Die oben beschriebenen Wachse lassen sich besonders gut granulieren.

Zur Herstellung der oben genannten Präparate der dekorativen Kosmetik sowie der pflegenden und reinigenden sowie der pharmazeutische Zubereitungen oder Hygieneartikel, beispielsweise von Inkontinenzprodukten, wird nach den an sich bekannten Verfahren vorgegangen, die dem Fachmann geläufig sind. Sie müssen gegenüber dem Stand der Technik nicht umgestellt werden.

Ein weiterer Gegenstand der vorliegenden Erfindung sind Präparate und Anwendungen der dekorativen Kosmetik, ausgewählt aus Lippenstiften, Gesichtspuder, Blushes, Lidschatten, Lidstrichstiften, Grundierungen, Make-up-Zubereitungen, Wimperntusche und Augenbrauenstiften, enthaltend die oben beschriebenen Polyethylenwachse mit guten organoleptischen Eigenschaften.

Ein weiterer Gegenstand der vorliegenden Erfindung sind Präparate und Anwendungen der pflegenden und reinigenden Kosmetik, ausgewählt aus Reinigungslotions, Handcremes, Handwaschpasten, Hautpflegemitteln, Intimpflegemitteln, Fußpflegemitteln, Lichtschutzmitteln, Repellents, Rasiermitteln, Haarentfernungsmitteln, Antiaknemitteln, Make-up, Nagelpflegemitteln, Kajalstiften, Seifen, Syndets, flüssigen Wasch-, Dusch-, und Badepräparaten, Cold Creams, Hautpflegeprodukte, Exfoliating Scrub Soaps, Gesichts-, Hand- oder Fuß-Peelings, Body-Scrubs, Gele und Lotionen, enthaltend die oben beschriebenen Polyethylenwachse mit verbesserten organoleptischen Eigenschaften.

Die erfindungsgemäßen kosmetischen Präparate und Anwendungen können z.B. eine Lösung, eine wasserfreie Zubereitung, eine Emulsion oder Mikroemulsion vom Typ Wasser-in-Öl oder vom Typ Öl-in-Wasser, eine multiple Emulsion, beispielsweise vom Typ Wasser-in-Ölin-Wasser, ein Gel, einen festen Stift, eine Salbe, ein Aerosol oder auch ein wässriges System bzw. eine Tensidzubereitung zur Reinigung von Haut und/oder Haaren, darstellen. Sie enthalten die oben beschriebenen Wachse in einem Anteil von 0,5 bis 20 Gew.-%.

Zusätzlich zu den oben beschriebenen Polyethylenwachsen können die erfindungsgemäßen kosmetischen Präparate und Anwendungen enthalten: Wirkstoffe wie beispielsweise UV-Filter, Parfüms und Riechstoffe, Emulgatoren, Tenside, feste Trägermaterialien wie beispielsweise Kreide, weitere Wachse wie beispielsweise Bienenwachs oder Carnaúbawachse, Vitamine, Basen oder Säuren, Alkohole sowie Wasser. Dabei kann es für verschiedene Präparate von Vorteil sein, mehrere verschiedene Emulgatoren, Wirkstoffe, Tenside, feste Trägermaterialien, Parfüme und Riechstoffe, Wachse, Basen oder Säuren, Vitamine oder Alkohole zu verwenden.

Die Herstellung der erfindungsgemäßen kosmetischen Präparate und Anwendungen erfolgt durch an sich bekannte Methoden. Bevorzugt ist das Mischen der Komponenten durch Verrühren. Das Mischen kann so erfolgen, dass sämtliche erforderlichen Komponenten in einem Schritt verrührt werden oder konsekutiv in mehreren Schritten. Dabei kann bei Raumtemperatur gearbeitet werden oder auch bei erhöhten Temperaturen bis über den Schmelzpunkt der oben beschriebenen Polyethylenwachse. Für einige Präparate wie beispielsweise Lippenstifte ist das Mischen bei Temperaturen oberhalb des Schmelzpunkts der Polyethylenwachse bevorzugt. Als höchste sinnvolle Temperatur für das Vermischen der Komponenten sei 150°C genannt. Bei der Herstellung derjenigen Anwendungen, für die eine sphärische Form des Polyethylenwachses erforderlich ist, wird das Mischen bei Temperaturen unterhalb des Schmelzpunktes des Polyethylenwachses durchgeführt.

Eine besondere Ausführungsform der vorliegenden Erfindung sind mikronisierte Polyethylenwachse mit verbesserten organoleptischen Eigenschaften sowie ein Verfahren zur Herstellung von mikronisierten Polyethylenwachsen. Nach der Polymerisation wird das in großtechnischen Anlagen üblicherweise als Grieß anfallende Wachs in einem nächsten Schritt erfindungsgemäß mikronisiert. Zum Mikronisieren können Mühlen verwendet werden, beispielsweise Luftstrahlmühlen. Die Wachse können aber auch gelöst oder aufgeschmolzen und in einem Sprühturm versprüht werden. Durch das Mikronisieren fällt das Wachs als sogenanntes Mikropulver oder Mikronisat an, wobei der Korndurchmesser 100 µm (Medianwert) nicht überschreitet, bevorzugt 50 µm und besonders bevorzugt 30 µm.

Die erfindungsgemäßen mikronisierten Polyethylenwachse haben eine Dichte von 0,8 bis 1,0 g/cm³, bevorzugt von 0,90 bis 0,96 g/cm³ und besonders bevorzugt von 0,93 bis 0,95 g/cm³, gemessen bei 23°C. Die Schmelzviskositäten liegen im Bereich von 20 bis 20000 cSt, bevorzugt im Bereich von 800 bis 2000 cSt, gemessen bei 120°C, das entspricht einem Molekulargewicht M_{w} von maximal 40.000 g, bevorzugt maximal 10.000 g und besonders bevorzugt maximal 7500 g. Die Molekulargewichtsverteilung liegt im Bereich von 2 bis 10. Die Schmelzpunkte liegen im Bereich von 60 bis 125°C, bevorzugt 80 bis 120°C. Der maximale Partikeldurchmesser beträgt nach der Mikronisierung, die durch Versprühen oder durch Mahlen erreicht wurde, maximal 30 µm.

Sowohl vor als auch nach der Mikronisierung zeigen die Wachse sehr gute organoleptische Eigenschaften, insbesondere sind sie Geruchs- und Geschmacks-neutral. Das ist insofern überraschend, weil sowohl die verwendeten Ketone und insbesondere Methylethylketon als auch die daraus unter Hochdruckbedingungen leicht entstehenden Dimere, Trimere und ähnliche Produkte einen charakteristischen und keinesfalls angenehmen Eigengeruch aufweisen.

Die Messung der organoleptischen Eigenschaften lässt sich apparativ, beispielsweise durch Gaschromatographie oder Differentialthermogravimetrie, bestimmen, wobei durch getrennte oder hintereinandergeschaltete Messapparaturen die Menge und die Art der entweichenden flüchtigen Verbindungen ermittelt wird. Von hoher Signifikanz sind insbesondere bei Mikronisaten die Tests durch Probandenteams.

Aufgrund ihrer sehr guten organoleptischen Eigenschaften eignen sich die erfindungsgemäßen Mikronisate vorzüglich zur Verwendung als Trägermaterialien bzw. Abrasiva für Zahnpasta oder kosmetische Anwendungen wie beispielsweise Lippenstifte, Rouge, Lidschatten und Cremes wie beispielsweise Handcremes oder Gesichtscremes.

Die Erfindung wird durch Arbeitsbeispiele erläutert.

### Arbeitsbeispiele:

### A) Herstellung der Polymerisate

Ethylen wurde in Anwesenheit des jeweiligen Molekulargewichtsreglers (Beispiele Nr. 1 bis 18) in einem Hochdruckautoklaven polymerisiert, wie er in der Literatur beschrieben wird (M. Buback et al., Chem. Ing. Tech. 1994, 66, 510.) Dazu wurden Monomer oder Monomerengemisch, dem als Initiator tert.-Butylperoxid und tert.-Butylperoxypivalat zugesetzt worden waren, sowie der Regler unter dem Reaktionsdruck von 1700 bar eingespeist. In Tabelle 1 sind die Polymerisationsbedingungen and analytische Daten der erhaltenen Polymerisate angegeben. Bei allen Versuchen wurde eine Reaktionstemperatur von 220°C und ein Reaktionsdruck von 1700 bar eingestellt.

**Tabelle 1: Polymerisationsbedingungen der Beispiele 1 - 9 und der Vergleichsbeispiele V1 V9.**

| Nr. | Regler | Regler | Ethylen | Ausbeute | r | h | Tₘₑₗₜ |
|---|---|---|---|---|---|---|---|
| | (Stoff) | [1/h] | [kg/h] | [kg PE/h] | [g/cm³] | mm²/s | °C |
| 1 | MEK | 2,35 | 12,2 | 2,0 | 0,9350 | 1080 | 112,8 |
| 2 | MEK | 2,70 | 12,1 | 1,8 | 0,9345 | 1880 | 112,9 |
| 3 | MEK | 2,65 | 12,0 | 1,9 | 0,9332 | 4110 | 113,0 |
| 4 | MIBK | 3,65 | 11,3 | 2,0 | 0,9348 | 870 | 110,6 |
| 5 | MIBK | 2,85 | 11,6 | 2,0 | 0,9344 | 1930 | 112,0 |
| 6 | MIBK | 2,15 | 12,2 | 1,9 | 0,9344 | 6530 | 112,0 |
| 7 | Aceton | 3,25 | 12,0 | 2,2 | 0,9340 | 1180 | 112,3 |
| 8 | Aceton | 2,70 | 12,3 | 2,2 | 0,9339 | 1830 | 112,9 |
| 9 | Aceton | 2,10 | 12,1 | 2,0 | 0,9328 | 4530 | 112,8 |
| V1 | PA | 0,34 | 11,6 | 1,5 | 0,9414 | 850 | 113,7 |
| V2 | PA | 0,24 | 11,4 | 1,5 | 0,9379 | 2070 | 113,6 |
| V3 | PA | 0,20 | 11,1 | 1,7 | 0,9372 | 4390 | 114,5 |
| V4 | IVA | 0,45 | 11,7 | 1,5 | 0,9256 | 4980 | 110,5 |
| V5 | IVA | 0,38 | 12,1 | 1,6 | 0,9245 | 2110 | 109,6 |
| V6 | IVA | 0,34 | 12,1 | 1,7 | 0,9254 | 4320 | 110,5 |
| V7 | C7 | 5,80 | 12,1 | 2,2 | 0,9256 | 4980 | 110,5 |
| V8 | C7 | 7,60 | 12,4 | 2,4 | 0,9245 | 2110 | 109,6 |
| V9 | C7 | 6,25 | 12,4 | 2,3 | 0,9254 | 4320 | 110,5 |

Verwendete Abkürzungen: MEK Methylethylketon, MIBK Methylisobutylketon, PA: Propionaldehyd, IVA: Isovaleraldehyd, C7: n-Heptan; PE: Polyethylenwachs.

### B) Organoleptische Prüfung der Polymerisate

Zur Prüfung der Wachse aus den oben aufgeführten Beispielen 1 bis 9 und V1 bis V9 wurden die Proben jeweils zwei Gruppen von Probanden vorgelegt. Probandenteam 1 bestand aus 23 Personen ohne besondere Vorbildung. Probandenteam 2 bestand aus 12 Personen, die professionell Geruchs- und Geschmacksproben durchführen. Die Benotung erfolgte jeweils mit Noten (1: sehr gut, 2: gut, 3: befriedigend, 4: ausreichend, 5 mangelhaft) und findet sich in Tabelle 2.

**Tabelle 2: Organoleptische Prüfung**

| Probe | Probandenteam 1 | Probandenteam 2 | Verwendeter Regler |
|---|---|---|---|
| 1 | 1 | 1 | MEK |
| 2 | 1 | 1-2 | MEK |
| 4 | 1-2 | 2 | MIBK |
| 5 | 2 | 1 | MIBK |
| 7 | 2 | 2 | Aceton |
| 8 | 2-3 | 2-3 | Aceton |
| V1 | 3, 5 | 5 | PA |
| V2 | 4 | 4 | PA |
| V4 | 3 | 3 , 5 | IVA |
| V5 | 4 | 3, 5 | IVA |
| V7 | 3-4 | 3-4 | C7 |
| V8 | 3-4 | 3 | C7 |

Die beschriebenen Proben 1 bis 9 sowie V1 bis V9 wurden in Peeling-Gels eingearbeitet, und es wurde nach einem Standardverfahren ein Lippenstift (in der Wärme, d.h. unter Aufschmelzen des Wachses) hergestellt. Die Peeling-Gels, hergestellt unter Verwendung der beschriebenen Proben 1 bis 9 waren den Vergleichs-Peeling-Gels in puncto Geruch deutlich überlegen, wobei die Peeling-Gels 1 und 2 am besten abschnitten.

### C) Anwendungsbeispiele

### C.1 Beispiel für die Formulierung eines Peeling-Gels:

| Gew.-% | Komponente |
|---|---|
| 0,5 | Vitamin-E-Acetat |
| 3,0 | Cremophor RH 410 |
| 15,0 | Ethanol |
| 0,01 | Uvinul® D 50 |
| 3,0 | D-Panthenol USP |
| 0,85 | Neutrol® TE |
| 60,0 | Carbopol® 940 (1%ig) |
| 5,0 | Polyethylenwachs aus Experiment 1 |

Das beschriebene Peeling-Gel wurde von den Probanden-Teams getestet und mit 1-2 bewertet. Ein analog hergestelltes Peeling-Gel, bei dem Polyethylenwachs aus Experiment V1 verwendet wurde, wurde von den Probanden-Teams mit 4 bzw. 5 bewertet.

### C.2 Beispiel für die Formulierung eines Lippenstifts:

In Lippenstiften, hergestellt unter Verwendung der Proben 1 bis 9, kam die Parfümierung wesentlich besser zur Wirkung als in den Vergleichsmustern, die unter Verwendung der Proben V1 bis V9 hergestellt wurden.

Lippenstift: Die Komponenten wurden in der Wärme, d.h. unter Aufschmelzen der Wachskomponenten, gemischt.

| Gew.-% | Komponente |
|---|---|
| 3,0 | Carnaúba-Wachs |
| 4,0 | Candelilla-Wachs |
| 2,0 | Bienenwachs |
| 7,0 | Mikrokristallines Wachs (Paraffinwachs) mit einem |
| | Schmelzpunkt 52-54°C |
| 1.5 | Cetylpalmitat |
| 6,0 | Petrolatum White |
| 4,0 | Lanolin-Wachs |
| 11,0 | Cetaryloctanoate |
| 2,0 | Polyethylen |
| 0,2 | Bisalbolol |
| 6,0 | Hydrierte Coco-Glyceride |
| 2,0 | Tocopheryl-Acetat |
| 0,5 | Tocopherol |
| 50,8 | Castor-Öl |

### C.3. Handcreme-Formulierungsbeispiel:

Handreiniger mit Reibkörper (alle Zahlenwerte in Gewichtsprozenten):

| | |
|---|---|
| Sulfopon^{®} HC Granulat | 13,70 |
| Glucopon^{®} 650 EC | 1,00 |
| Dehyton^{®} K | 1,2 |
| Texapon^{®} NSO | 1,00 |
| Comperlan^{®} KD | 0,50 |
| Kreide | 32,40 |
| Polyethylenwachs | 8,10 |
| Soda | 0,90 |
| Edenor^{®} HT 35 | 1,10 |
| NaOH (50%ig) | 0,30 |
| Zitronensäure | 3,00 |
| Parfümöl | 0,30 |
| VE-Wasser | zu 100 auffüllen |
| pH-Wert: 5,5-6,0 | |

Es wurden verschiedene Handreiniger mit den Polyethylenwachs-Proben 1, 4, 5, V1 und V9 hergestellt und von den Probendenteams wie folgt beurteilt:

| Eingesetztes Wachs | Probanden-Team 1 | Probanden-Team 2 |
|---|---|---|
| 1 | 1-2 | 1 |
| 4 | 3 | 3 |
| 5 | 3 | 3-4 |
| V1 | 4-5 | 5 |
| V9 | 5 | 5 |

### D) Herstellung und organoleptische Prüfung der Mikronisate

Die in A) anfallenden Polymerisate wurden im Anschluss an die Polymerisation mikronisiert. Die Mikronisierung erfolgte jeweils auf einer Luftstrahlmühle der Fa. Micro-Macinazione, Typ Chrispro-Jetmill MC 100, auf eine Partikelgröße von 10 µm (Median). Man erhielt Mikronisate.

Zur Prüfung der Wachse aus den oben aufgeführten Beispielen 1 bis 9 und V1 bis V9 wurden die Proben den Probandenteams aus A) vorgelegt. Die Beurteilung findet sich in Tabelle 3.

**Tabelle 3: Organoleptische Prüfung der Mikronisate**

| Probe | Probandenteam 1 | Probandenteam 2 | Verwendeter Regler |
|---|---|---|---|
| 1 | 1 | 1 | MEK |
| 2 | 1 | 1-2 | MEK |
| 4 | 1-2 | 2 | MIBK |
| 5 | 2 | 1 | MIBK |
| 7 | 2 | 2 | Aceton |
| 8 | 2-3 | 2-3 | Aceton |
| V1 | 3,5 | 5 | PA |
| V2 | 4 | 4 | PA |
| V4 | 3 | 3,5 | IVA |
| V5 | ' 4 | 3,5 | IVA |
| V7 | 3-4 | 3-4 | C7 |
| V8 | 3-4 | 3 | C7 |

Die beschriebenen Proben 1 bis 9 sowie V1 bis V9 wurden in Zahnpasten eingearbeitet, und es wurde nach einem Standardverfahren eine kosmetische Seife hergestellt. Die Zahnpasten, hergestellt unter Verwendung der beschriebenen Proben 1 bis 9 waren den Vergleichszahnpasten in puncto Geruch deutlich überlegen, wobei die Pasten 1 und 2 am besten abschnitten.

### Formulierung einer Zahnpasta:

| Bestandteile (Angaben in Ges.-%) (INCI-Namen) | Beispiel A | Beispiel B |
|---|---|---|
| Wasser | 39,7 | 39,7 |
| Sorbitol | 35,0 | 35,0 |
| Wasserhaltiges Kieselgel | 0,0 | 0,0 |
| Luvitol® PE 10 M | 20,0 | 20,0 |
| Natrium-Carboxymethylcellulose | 1,6 | 1,6 |
| Saccharin | 0,2 | 0, 2 |
| TiO₂ | 0,5 | 0,5 |
| Natrium-Monofluorophosphat | 1,0 | 1,0 |
| Aromaöl | 0,5 | 0,5 |
| Kieselgel | 1,5 | 1,5 |

Sämtliche Bestandteile wurde bei Raumtemperatur homogenisiert.

Beispiel A wurde unter Verwendung von mikronisiertem Wachs, Probe 1, hergestellt, Beispiel B unter Verwendung von Probe V2. Während Zahnpasta (Beispiel A) einen erfrischenden Geruch verströmte, hatte Zahnpasta des Beispiels B eine Geruchskomponente, die mehrere Mitglieder des Probandenteams 1 als äußerst unangenehm empfanden.

In Lippenstiften, hergestellt unter Verwendung der Proben 1 bis 9, kam die Parfümierung wesentlich besser zur Wirkung als in den Vergleichsmustern, die unter Verwendung der Proben V1 bis V9 hergestellt wurden.

Beispiel für die Formulierung eines Lippenstifts:

| Lippenstift | |
|---|---|
| Gew.-% | Komponente |
| 3,0 | Carnaúba-Wachs |
| 4,0 | Candelilla-Wachs |
| 2,0 | Bienenwachs |
| 7,0 | Mikrokristallines Wachs (Paraffinwachs) mit einem |
| | Schmelzpunkt 52-54°C |
| 1.5 | Cetylpalmitat |
| 6,0 | Petrolatum White |
| 4,0 | Lanolin-Wachs |
| 11,0 | Cetaryloctanoate |
| 2,0 | mikronisiertes Polyethylen |
| 0,2 | Bisalbolol |
| 6,0 | Hydrierte Coco-Glyceride |
| 2,0 | Tocopheryl-Acetat |
| 0,5 | Tocopherol |
| 50,8 | Castor-Öl |

Die mit Ketonen, insbesondere mit Methylethylketon oder Cyclohexanon als Regler hergestellten Polyethylenwachse eignen sich gleichfalls vorzüglich für geruchsfreie Peeling-Gels. Beispiel für die Formulierung eines Peeling-Gels:

## Patentansprüche

1. Kosmetische Präparate, enthaltend 0,5 bis 20 Gew.-% Polyethylenwachse, hergestellt durch (Co-)Polymerisation von Ethylen bei Drücken von 400 bis 4000 bar und Temperaturen im Bereich von 180 bis 350°C unter Verwendung eines oder mehrerer aliphatischer oder alicyclischer Ketone als Molekulargewichtsregler.

2. Kosmetische Präparate nach Anspruch 1, enthaltend Polyethylenwachse mit einem maximalen Molekulargewicht M_{w} von 40.000 g.

3. Präparate der dekorativen Kosmetik, ausgewählt aus Lippenstiften, Gesichtspuder, Blushes, Lidschatten, Lidstrichstiften, Grundierungen, Make-up-zubereitungen, Wimperntusche und Augenbrauenstiften, nach Anspruch 1 und 2.

4. Präparate der pflegenden und reinigenden Kosmetik, ausgewählt aus Reinigungslotions, Handcremes, Handwaschpasten, Hautpflegemitteln, Intimpflegemitteln, Fußpflegemitteln, Lichtschutzmitteln, Repellents, Rasiermitteln, Haarentfernungsmitteln, Antiaknemitteln, Make-up, Nagelpflegemitteln, Kajalstiften, Seifen, Syndets, flüssigen Wasch-, Dusch-, und Badepräparaten, Cold Creams, Hautpflegeprodukte, Exfoliating Scrub Soaps, Gesichts-, Hand- oder Fuß-Peelings, Body-Scrubs, Gele und Lotionen, nach Anspruch 1 und 2.

5. Verfahren zur Herstellung von kosmetischen Präparaten nach den Ansprüchen 1 bis 4, **dadurch gekennzeichnet, dass** man 0,5 bis 20 Gew.-% Polyethylenwachs bei 20 bis 200°C mit weiteren Stoffen, ausgewählt aus Wirkstoffen, Parfüms und Riechstoffen, Emulgatoren, Tensiden, festen Trägermaterialien, weiteren Wachsen, Vitaminen, Basen oder Säuren, Alkoholen sowie Wasser mischt.

6. Verfahren nach Anspruch 5, **dadurch gekennzeichnet, dass** man Wirkstoffe wählt aus UV-Filtern,

7. Verwendung von Polyethylenwachsen gemäß den Ansprüchen 1 und 2 für kosmetische Präparate.

8. Verwendung von mikronisierten Polyethylenwachsen mit einem Korndurchmesser (Medianwert) von maximal 100 µm, hergestellt durch (Co-)Polymerisation von Ethylen unter Hochdruckbedingungen unter Verwendung eines oder mehrerer aliphatischer oder alicyclischer Ketone als Molekulargewichtsregler und anschließende Mikronisierung, für kosmetische Präparate und als Träger für Zahnpasta.

9. Verwendung von mikronisierten Polyethylenwachsen mit einem Korndurchmesser (Medianwert) von maximal 100 µm, hergestellt durch (Co-)Polymerisation von Ethylen unter Hochdruckbedingungen unter Verwendung eines oder mehrerer aliphatischer oder alicyclischer Ketone als Molekulargewichtsregler und anschließende Mikronisierung, für Lidschatten, Lippenstifte oder Rouge.

10. Verwendung nach Anspruch 8 oder 9, **dadurch gekennzeichnet, dass** mikronisierte Polyethylenwachse unter Verwendung von Methylethylketon oder Cyclohexanon als Molekulargewichtsregler hergestellt werden.

11. Verwendung nach einem der Ansprüche 8 bis 10, **dadurch gekennzeichnet, dass** mikronisierte Polyethylenwachse ein maximales Molekulargewicht M_{w}, von 40.000 g aufweisen.

12. Verwendung nach einem der Ansprüche 8 bis 11, **dadurch gekennzeichnet, dass** mikronisierte Polyethylenwachse einen maximalen Korndurchmesser von 30 µm aufweisen.

## Claims

1. A cosmetic preparation, comprising 0.5 to 20% by weight of polyethylene waxes prepared by (co)polymerization of ethylene at pressures of from 400 to 4000 bar and temperatures in the range from 180 to 350°C using one or more aliphatic or alicyclic ketones as molecular weight regulator.

2. The cosmetic preparation according to claim 1, comprising polyethylene waxes with a maximum molecular weight M_{w} of 40 000 g.

3. A preparation of decorative cosmetics, selected from lipsticks, face powders, blushers, eyeshadows, eyeliner pencils, foundations, make-up preparations, mascara and eyebrow pencils, according to claims 1 and 2.

4. A preparation of care and cleansing cosmetics selected from cleansing lotions, handcreams, handwashing pastes, skincare compositions, intimate care compositions, footcare compositions, sunscreen compositions, repellents, shaving compositions, hair-removal compositions, antiacne compositions, make-up, nailcare compositions, kohl pencils, soaps, syndets, liquid washing, showering and bath preparations, cold creams, skincare products, exfoliating scrub soaps, face, hand or foot peels, body scrubs, gels and lotions, according to claims 1 and 2.

5. A process for the preparation of cosmetic preparations according to claims 1 to 4, which comprises mixing 0.5 to 20% by weight of polyethylene wax at 20 to 200°C with further substances selected from active ingredients, perfumes and fragrances, emulsifiers, surfactants, solid carrier materials, further waxes, vitamins, bases or acids, alcohols and water.

6. The process according to claim 5, wherein active ingredients are selected from UV filters.

7. The use of polyethylene waxes according to claims 1 and 2 for cosmetic preparations.

8. The use of micronized polyethylene waxes having a particle diameter (median value) of at most 100 µm, prepared by (co)polymerization of ethylene under high-pressure conditions using one or more aliphatic or alicyclic ketones as molecular weight regulator and subsequent micronization, for cosmetic preparations and as carriers for toothpaste.

9. The use of micronized polyethylene waxes having a particle diameter (median value) of at most 100 µm, prepared by (co)polymerization of ethylene under high-pressure conditions using one or more aliphatic or alicyclic ketones as molecular weight regulator and subsequent micronization, for eyeshadows, lip pencils or blusher.

10. The use according to claim 8 or 9, wherein micronized polyethylene waxes are prepared using methyl ethyl ketone or cyclohexanone as molecular weight regulator.

11. The use according to any of claims 8 to 10, wherein micronized polyethylene waxes have a maximum molecular weight M_{w} of 40 000 g.

12. The use according to any of claims 8 to 11, wherein micronized polyethylene waxes have a maximum particle diameter of 30 µm.

## Revendications

1. Préparations cosmétiques, contenant de 0,5 à 20 % en poids de cires de polyéthylène préparées par (co-)polymérisation d'éthylène sous des pressions de 400 à 4 000 bars et à des températures dans la plage de 180 à 350 °C, avec utilisation d'une ou plusieurs cétones aliphatiques ou alicycliques en tant que régulateurs de masse moléculaire.

2. Préparations cosmétiques selon la revendication 1, contenant des cires de polyéthylène ayant une masse moléculaire maximale M_{w} de 40 000 g.

3. Préparations pour la cosmétique de maquillage, choisies parmi les bâtons pour les lèvres, les poudres pour le visage, les rouges à joues, les ombres à paupières, bâtons ligneurs à paupières, les fonds de teint, les préparations de maquillage, les produits pour les cils et crayons à sourcils, selon les revendications 1 et 2.

4. Préparations pour la cosmétique de soin et de nettoyage, choisies parmi les lotions de nettoyage, les crèmes pour les mains, les pâtes lavantes pour les mains, les produits pour le soin de la peau, les produits de soin intime, les produits pour le soin des pieds, les produits de protection solaire, les insectifuges, les produits de rasage, les produits épilatoires, les produits anti-acnéiques, les produits de maquillage, les produits pour le soin des ongles, les crayons khôl, les savons, les détergents synthétiques, les préparations liquides pour la toilette, la douche et le bain, les crèmes de beauté, les produits pour le soin de la peau, les savons exfoliants, les gommages pour le visage, les mains ou les pieds, les body-scrubs, les gels et lotions, selon la revendication 1 ou 2.

5. Procédé pour la production de préparations cosmétiques selon les revendications 1 à 4, **caractérisé en ce qu'**on mélange 0,5 à 20 % en poids de cire de polyéthylène, à une température de 20 à 200 °C, avec d'autres substances choisies parmi des substances actives, des parfums et des substances odorantes, des émulsifiants, des tensioactifs, des matières solides jouant le rôle de véhicule, d'autres cires, des vitamines, des bases ou des acides, des alcools ainsi que de l'eau.

6. Procédé selon la revendication 5, **caractérisé en ce qu'**on choisit les substances actives parmi les filtres UV.

7. Utilisation de cires de polyéthylène selon les revendications 1 et 2, pour des préparations cosmétiques.

8. Utilisation de cires de polyéthylène micronisées ayant un diamètre de grain (valeur moyenne) d'au maximum 100 µm, préparées par (co-)polymérisation d'éthylène dans des conditions de pression élevée, avec utilisation d'une ou plusieurs cétones aliphatiques ou alicycliques en tant que régulateurs de masse moléculaire et micronisation subséquente, pour des préparations cosmétiques et en tant que véhicule pour pâte dentifrice.

9. Utilisation de cires de polyéthylène micronisées ayant un diamètre de grain (valeur moyenne) d'au maximum 100 µm, préparées par (co-)polymérisation d'éthylène dans des conditions de pression élevée, avec utilisation d'une ou plusieurs cétones aliphatiques ou alicycliques en tant que régulateurs de masse moléculaire et micronisation subséquente, pour des ombres à paupières, des bâtons pour les lèvres ou des bâtons de rouge.

10. Utilisation selon la revendication 8 ou 9, **caractérisée en ce que** les cires de polyéthylène micronisées sont préparées avec utilisation de méthyléthylcétone ou de cyclohexanone en tant que régulateurs de masse moléculaire.

11. Utilisation selon l'une quelconque es revendications 8 à 10, **caractérisée en ce que** les cires de polyéthylène micronisées présentent une masse moléculaire moyenne M_{w} de 40 000 g.

12. Utilisation selon l'une quelconque es revendications 8 à 11, **caractérisée en ce que** les cires de polyéthylène micronisées présentent un diamètre maximum de grain de 30 µm.
